# EUROPÄISCHE PATENTSCHRIFT

(11) **EP 0 539 423 B1**
(45) Veröffentlichungstag und Bekanntmachung des Hinweises auf die Patenterteilung: **21.12.1994**
(21) Anmeldenummer: 91912729.0
(22) Anmeldetag: 09.07.1991
(51) Int. Cl.: A61K 7/13

(54) **HAARFÄRBEMITTEL-ZUBEREITUNG**
HAIR DYEING COMPOSITION
COMPOSITION DE TEINTURE DES CHEVEUX

(30) Priorität: 18.07.1990 DE 4022848
(43) Veröffentlichungstag der Anmeldung: 05.05.1993
(73) Patentinhaber: Henkel Kommanditgesellschaft auf Aktien, 40191 Düsseldorf (DE)
(72) Erfinder: HÖFFKES, Horst, D-4000 Düssseldorf-Hellerhof (DE); SCHRADER, Dieter, D-4000 Düsseldorf 13 (DE); SCHETTIGER, Norbert, D-4010 Hilden (DE); JESCHKE, Rainer, D-4000 Düsseldorf 13 (DE)
(86) Internationale Anmeldenummer: EP9101274
(87) Internationale Veröffentlichungsnummer: WO9201438

(56) Entgegenhaltungen:
- EP-A- 0 188 216
- WO-A-90/00386

## Beschreibung

Die Erfindung betrifft Haarfärbemittel-Zubereitungen, bestehend aus einer fließfähigen wäßrigen Oxidationsfarbstoffzubereitung (A) und einer fließfähigen, wäßrigen Oxidationsmittelzubereitung (B), die unmittelbar vor dem Aufbringen auf das Haar zu einem gelförmigen Färbeansatz vermischt werden, und die nach dem Vermischen als strukturviskoses, fließfähiges Gel vorliegen.

Als Träger für Oxidationshaarfärbemittel werden überwiegend O/W-Cremes oder Gele verwendet. Die Oxidationsfarbstoffzubereitungen sollen nach Zugabe des Oxidationsmittels, meist einer wäßrigen Wasserstoffperoxid-Zubereitung, eine cremige oder viskos gelförmige Konsistenz aufweisen, die ein leichtes Auftragen und Verteilen auf dem Haar, z.B. mit einem Pinsel, und eine gewisse Haftung auf dem Haar ermöglicht, ohne daß das Färbemittel vom Haar abläuft und Kopfhaut oder Gesicht benetzt.

Oxidationsfarbstoffzubereitungen vom Gel-Typ weisen als Träger üblicherweise Seifen im Gemisch mit Wasser und niederen Alkoholen oder Glycolen auf. Solche Zubereitungen ergeben nach dem Vermischen mit wäßrigen Oxidationsmittel-Lösungen viskose, gelförmige Färbeansätze. Nachteilig ist die Bildung von Kalkseifen beim Ausspülen des Färbemittels mit hartem Wasser. Ein weiterer Nachteil besteht darin, daß bei einem hohen Seifenanteil die Farbstoff-Zubereitung zu viskos wird, bei einem niedrigeren Seifenanteil aber der Färbeansatz nicht die erforderliche Viskosität erreicht. Auch die Mitverwendung eines synthetischen Tensids mit einer die Kalkseife dispergierenden Wirkung stört die Ausbildung der fließfähigen Gelstruktur des Färbesansatzes. Es bestand daher die Aufgabe ein für die Oxidationsmittel-Zubereitung geeignetes Trägersystem zu finden, das die genannten Probleme nicht oder in erheblich geringerem Ausmaß aufweist.

Es wurde gefunden, daß Haarfärbemittel, bestehend aus einer fließfähigen, wäßrigen Oxidationsfarbstoffzubereitung (A) und einer fließfähigen, wäßrigen Oxidationsmittelzubereitung (B), die unmittelbar vor dem Aufbringen auf das Haar miteinander im Gewichtsverhältnis A:B = 1:2 bis 2:1 zu einem gelförmigen Färbeansatz vermischt werden, dann besonders gute Anwendungseigenschaften aufweisen, wenn die Oxidationsfarbstoffzubereitung (A) als Trägerkomponenten
eigenschaften aufweisen, wenn die Oxidationsfarbstoffzubereitung (A) als Trägerkomponenten
0,5 bis 5 Gew.-% einer flüssigen Fettsäure mit 16 bis 22 C-Atomen in Form einer wasserlöslichen Seife,
1 bis 5 Gew.-% eines Polyols mit 2 bis 6 C-Atomen,
0,5 bis 5 Gew.-% eines wasserlöslichen, synthetischen Tensids und zur Verdickung
1 bis 10 Gew.-% eines Anlagerungsproduktes von 1 bis 5 Mol Ethylenoxid an einen linearen Fettalkohol mit 12 bis 22 C-Atomen und/oder
1 bis 6 Gew.-% eines Anlagerungsproduktes von 1 bis 4 Mol Ethylenoxid an ein lineares Fettalkylamin mit 12 bis 22 C-Atomen und gegebenenfalls
0 bis 10 Gew.-% einer Verbindung der Formel (I)

R¹-(OC₂H₄)ₓ-A-(C₂H₄O)_{y}-R² (I)

in der R¹ und R² lineare Alkyl- oder Alkenylgruppen mit 12 bis 22 C-Atomen, x und y = 0 oder 1 und A ein Sauerstoffatom oder eine Gruppe HO-C₂H₄-N〈ist, enthält.

Die zur Seifenbildung geeigneten Fettsäuren sind bevorzugt flüssige oder niedrigschmelzende ungesättigte lineare C₁₆-C₂₂-Fettsäuren wie Palmitoleinsäure, Ölsäure, Elaidinsäure, Petroselinsäure, Petroselaidinsäure, Gadoleinsäure, Erucasäure, Brassidinsäure sowie Gemische dieser Fettsäuren untereinander und mit geringeren Anteilen gesättigter linearer Fettsäuren mit 12 bis 22 C-Atomen. Andere ebenfalls geeignete Fettsäuren sind verzweigte Fettsäuren mit 16 bis 22 C-Atomen, z.B. die 2-Hexyl-decansäure und die 2-Octyl-dodecansäure.

Zur Überführung der Fettsäuren in wasserlösliche Seiten eignen sich Alkalihydroxide und Alkalicarbonate, Ammoniak sowie Mono-, Di- und Trialkanolamine mit 2 bis 4 C-Atomen in der Alkanolgruppe.

Als Polyole mit 2 bis 6 C-Atomen eignen sich z.B. Ethylenglycol, 1,2-Propylenglycol, 1,3-Propylenglycol, Glycerin, Erythrit, Trimethylolpropan, Diethylenglycol und Dipropylenglycol. 1,2-Propylenglycol ist bevorzugt.

Als wasserlösliche synthetische Tenside eignen sich bevorzugt anionische, amphotere, zwitterionische und nichtionische Tenside mit guter Wasserlöslichkeit und mit gutem Kalkseifendispergiervermögen. Solche Tenside weisen in der Regel eine lipophile lineare Alkyl- oder Acylgruppe mit 12 bis 18 C-Atomen und eine stark dissoziierte ionische Gruppe oder eine nichtionische wasserlöslichmachende Polyethergruppe auf. Geeignet sind z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen. Weitere geeignete Aniontenside sind z.B. lineare Alkansulfonate und α-Olefinsulfonate mit 12 bis 18 C-Atomen. Geeignete amphotere Tenside sind z.B. N-Fettalkyl-dimethyl-glycin oder N-Fettalkylaminopropionsäure. Geeignete zwitterionische Tenside sind z.B. N-Fettalkyl-dimethylammonium-glycinat oder N-Fettacylaminopropyldimethylglycinat. Geeignete nichtionogene Tenside sind z.B. die Anlagerungsprodukte von 7 bis 30 Mol Ethylenoxid an lineare Fettalkohole mit 12 bis 18 C-Atomen, an Fettsäuren mit 12 bis 18 C-Atomen sowie an Fettsäuremonoglyceride und an Fettsäure-sorbitanmonoester. Andere geeignete nichtionogene Tenside sind die Fettalkylaminoxide und die Fettalkyl-glucoside. Die Fettalkylgruppe kann in den genannten Produkten 12 bis 18 C-Atome aufweisen.

Es hat sich gezeigt, daß durch den Zusatz weiterer, begrenzt wasserlöslicher, nichtionogener Tenside eine Verdickung der Oxidationsfärbemittel-Zubereitung und insbesondere des unmittelbar vor der Anwendung hergestellten Färbeansatzes erreicht wird.

Als Anlagerungsprodukte von 1 bis 5 Mol Ethylenoxid an linearen Fettalkohol mit 12 bis 22 C-Atomen eignen sich alle nach den bekannten technischen doch die Anlagerungsprodukte an ungesättigte C₁₆-C₁₈-Fettalkohole, insbesondere an Oleylalkohol oder Oleylalkohol enthaltende Fettalkoholfraktionen. Noch mehr bevorzugt sind die Anlagerungsprodukte, die nur wenig freien Fettalkohol enthalten und eine eingeengte Homologenverteilung aufweisen (sogenannte "narrow range ethoxylates"), wie sie z.B. nach dem in DE 38 43 713 A1 beschriebenen Verfahren zugänglich sind.

Als Anlagerungsprodukte von 1 bis 4 Mol Ethylenoxid an ein lineares Fettalkylamin mit 12 bis 22 C-Atomen eignen sich alle nach bekannten technischen Verfahren zugänglichen Addukte, die auch im Handel erhältlich sind. Besonders geeignet ist das Anlagerungsprodukt von 2 Mol Ethylenoxid an ein C₁₂-C₁₈-Kokosalkylamin.

Geeignete Verbindungen der Formel

R¹-(OC₂H₄)ₓ-A-(C₂H₄O)_{y}-R² (I)

in welcher A ein Sauerstoffatom ist, sind Dialkylether mit 12 bis 18 C-Atomen in den Alkylgruppen. Solche Produkte sind literaturbekannt. Noch besser geeignet sind die Produkte der Formel I, in welchen x oder y oder beide = 1 sind. Solche Di-alkyloxethyl-ether lassen sich durch literaturbekannte Veretherungsverfahren aus Fettalkoholen und Fettalkyl-oxyethanolen herstellen. Die Produkte der Formel I, in welchen A eine Gruppe HO-C₂H₄N〈 ist, lassen sich z.B. aus Triethanolamin durch O-Alkylierung mit 2 Mol eines Schwefelsäurehalbestersalzes eines C₁₂-C₂₂-Fettalkohols nach dem in DE 35 04 242 beschriebenen Verfahren zur Herstellung von Etheraminen gewinnen.

Besonders bevorzugte Verbindungen der Formel I sind z.B. Di-cetyl-/stearylether, Di-cetyl-/stearyl-oxyethyl-ether und N,N-Bis-(2-cetyl-/stearyloxyethyl)-aminoethanol.

Während mit Anlagerungsprodukten von 1 bis 5 Mol Ethylenoxid an einen linearen Fettalkohol die erforderliche Verdickung meist erreicht wird, ist es bei Einsatz der Anlagerungsprodukte von 1 bis 4 Mol Ethylenoxid an ein lineares Fettalkylamin gelegentlich vorteilhaft, diese in Kombination mit 1 bis 10 Gew.-% einer Verbindung der Formel I zu verwenden. Es wird dabei lineares Fettalkylamin gelegentlich vorteilhaft, diese in Kombination mit 1 bis 10 Gew.-% einer Verbindung der Formel I zu verwenden. Es wird dabei angestrebt, daß die Oxidationsfärbemittel-Zubereitung (A) eine Viskosität von wenigstens 1 Pa.s (20 °C) und der Färbeansatz, der durch Vermischen von (A) mit der Oxidationsmittelzubereitung (B) entsteht, eine Viskosität von wenigstens 10 Pa.s (20 °C) aufweist (Viskositätsmessung mit dem Rotationsviskosimeter Brookfield, Typ RTV, Spindel 4, 4 UpM).

Darüber hinaus sind in der Oxidationsfärbemittel-Zubereitung (A) natürlich die Oxidationsfarbstoffvorprodukte, die in Gegenwart von Oxidationsmitteln den Farbstoff ausbilden, sowie gegebenenfalls auch Direktfarbstoffe enthalten. Als Oxidationsfarbstoffvorprodukte eignen sich z.B. die bekannten Farbbasen bzw. Entwicklerverbindungen und bekannte Modifikatoren bzw. Kupplerverbindungen. Die Oxidationsfarbstoffe bilden sich durch oxidative Kupplung einer oder mehrerer Entwicklerkomponenten untereinander oder mit einer oder mehreren Kupplerkomponenten in Gegenwart eines Oxidationsmittels aus. Als Entwicklersubstanzen werden üblicherweise primäre aromatische Amine mit einer weiteren in Para- oder Orthoposition befindlichen freien oder substituierten Hydroxy- oder Aminogruppe, Diaminopyridin-Derivate, heterocyclische Hydrazonderivate, 4-Aminopyrazolonderivate oder Tetraaminopyrimidine eingesetzt. Als sogenannte Kupplersubstanzen werden z.B. m-Phenylendiaminderivate, Phenole, Naphthole, Resorcinderivate oder Pyrazolone verwendet.

Darüber hinaus können die Oxidationsfärbemittel-Zubereitungen (A) geeignete Zusätze zur Stabilisierung der Farbstoffvorprodukte enthalten; dies sind Komplexbildner, z.B. Ethylendiaminotetraessigsäure, Nitrilotriessigsäure, 1-Hydroxyethan-1,1-diphosphonsäure oder andere Organodiphosphonsäuren in Form ihrer Alkalisalze, Antioxidantien, wie z.B. Natriumsulfit, Natriumbisulfit, Hydrochinon oder Salze der Thioglycolsäure oder Ascorbinsäure, Puffersalze, wie z.B. Ammoniumsulfat, Ammoniumcarbonat, Ammoniumcitrat sowie Ammoniak oder ein Alkanolamin zur Einstellung eines pH-Wertes von 8 bis 10. Weiterhin können haarkosmetische Hilfsstoffe wie z.B. wasserlösliche kationische Polymere, mit Seifen verträgliche Kationtenside und wasserlösliche Proteine oder Proteinabbauprodukte enthalten sein.

Die Oxidationsmittelzubereitung (B) enthält bevorzugt
3 bis 10 Gew.-% Wasserstoffperoxid
0,1 bis 5 Gew.-% eines wasserlöslichen, synthetischen Tensids und
1 bis 5 Gew.-% eines dispergierten Acrylsäure- und/oder Methacrylsäure-Polymerisats oder -Copolymerisats
im wäßrigen Träger neben den in solchen Zubereitungen üblichen Stabilisierungshilfsmitteln.

Als wasserlösliche synthetische Tenside können die bereits für die Oxidationsfärbemittel-Zubereitung genannten anionischen, amphoteren, zwitterionischen und nichtionischen Tenside oder Gemische davon verwendet werden. Bevorzugt werden anionische Tenside, z.B. Schwefelsäurehalbestersalze von linearen Fettalkoholen mit 12 bis 18 C-Atomen oder von Fettalkoholpolyglycolethern mit 12 bis 16 C-Atomen in der Alkylgruppe und 1 bis 10 Glycolethergruppen in Form ihrer Alkali-, Magnesium-, Ammonium- oder Alkanolammoniumsalze eingesetzt.

Besonders günstig für den Viskositätsaufbau nach dem Vermischen von Oxidationsfärbemittelzubereitung (A) und Oxidationsmittelzubereitung (B) ist der Gehalt an dispergierten Acrylsäure- und/oder Methacrylsäure-Polymerisat oder -Copolymerisat in (B).

Solche Dispersionen von Copolymerisaten, z.B. aus wenigstens 10 Gew.-% Acrylsäure-Niedrigalkylester, 25 bis 70 Gew.-% Methacrylsäure und ggf. 0 bis 40 Gew.-% eines weiteren Comonomeren, sind z.B. in GB 870 994 beschrieben. Aus DE 11 64 095 sind Mischpolymerisate aus 50 bis 75 Gew.-% Ethylacrylat, 25 bis 35 Gew.-% Acrylsäure und 0 bis 25 Gew.-% anderer Comonomerer bekannt. Geeignete Dispersionen dieser Art sind im Handel erhältlich, z.B. unter der Handelsbezeichnung Latekoll ^{(R)}D (BASF). Besonders gut eignen sich die in DE 34 45 549 beschriebenen Copolymerisate aus 50 bis 60 Gew.-% Ethylacrylat, 30 bis 40 Gew.-% Methacrylsäure und 5 bis 15 Gew.-% Acrylsäure, vernetzt mit Ethylenglycoldimethacrylat.

Die Oxidationsmittelzubereitung (B) enthält außerdem bevorzugt Komplexbildner und Puffersalze zur Einstellung eines pH-Wertes von 2 bis 5. In diesem schwach sauren Medium bleiben die Acrylsäure- und/oder Methacrylsäure-Polymerisat-Dispersionen dünnflüssig und stabil. Beim Vermischen mit der alkalischen Oxidationsfärbemittel-Zubereitung (A), die Ammoniak und Puffersalze zur Einstellung eines pH-Wertes von 8 bis 10 enthält, steigt der pH-Wert der Mischung an und die Carboxylgruppen des Polymerisats oder Copolymerisats werden in die Salzform überführt. Dabei beginnen die Polymerisate sich unter Entknäuelung der Polymerketten im wäßrigen Medium zu lösen und die Viskosität der Lösung anzuheben.

Die folgenden Beispiele sollen den Gegenstand der Erfindung näher erläutern:

### Beispiele

Zur Herstellung der Beispiele wurden folgende Komponenten verwendet:
(1) Farbstofflösung

| | |
|---|---|
| p-Phenylendiamin | 0,2564 g |
| Resorcin | 0,2366 g |
| p-Aminophenol · HCl | 0,0585 g |
| 2,4-Dichlor-m-aminophenol · HCl | 0,0347 g |
| 4-Chlorresorcin | 0,1053 g |
| Ammoniak (konz., ca. 25 Gew.-% in Wasser) | 1,0 g |
| Wasser | ad 7,0 g |

(2) Stabilisatorlösung

| | |
|---|---|
| 1-Hydroxyethan-1,1-diphosphonsäure | 0,2 g |
| (NH₄)₂ SO₄ | 0,75 g |
| Na₂SO₃ | 0,5 g |
| Na-Ascorbat | 0,5 g |
| Ammoniak (konz., ca. 25 Gew.-% in Wasser) | 1,0 g |
| Wasser | ad 8,0 g |

(3) Texapon N: 28 gew.-%ige Lösung von Fettalkohol(C₁₂-C₁₄)-polyglycol(2 EO)ethersulfat, Na-Salz in Wasser
(4) Dehyton K: 30 gew.-%ige Lösung von N-Kokosacyl(C₁₂-C₁₈)-aminopropyl-N,N-dimethylammoniumglycinat in Wasser
(5) K 20: Addukt von 2 Mol Ethylenoxid an Kokosalkyl(C₁₂-C₁₈)-amin
(6) DTE: Di-cetyl-/stearylether
(7) DTEE: Di-cetyl-/stearyloxyethyl-ether
(8) Arlypon F: Addukt von 2,5 Mol Ethylenoxid an C₁₂-C₁₄-Fettalkohol

### Zusammensetzung der Oxidationsfarbstoff-Zubereitungen (A)

**Tabelle I**

| | 1 | 2 | 3 | 4 | 5 |
|---|---|---|---|---|---|
| Ölsäure | 1,5 g | 1,5 g | 1,5 g | 1,5 g | 1,5 g |
| Texapon N (3) | 4,0 g | 4,0 g | 4,0 g | 4,0 g | 4,0 g |
| Dehyton K (4) | 3,0 g | 3,0 g | 3,0 g | 3,0 g | 3,0 g |
| 1,2-Propylenglycol | 2,15 g | 2,15 g | 2,15 g | 2,15 g | 2,15 g |
| Arlypon F (8) | 5,4 g | 7,4 g | 5,4 g | - | - |
| K 20 (5) | - | - | 2,0 g | 2,0 g | 2,0 g |
| DTE (6) | - | - | - | 5,4 g | - |
| DTEE (7) | - | - | - | - | 5,4 g |
| Farbstofflösung (1) | 7,0 g | 7,0 g | 7,0 g | 7,0 g | 7,0 g |
| Stabilisatorlösung (2) | 8,0 g | 8,0 g | 8,0 g | 8,0 g | 8,0 g |
| Parfümöl | 0,2 g | 0,2 g | 0,2 g | 0,2 g | 0,2 g |
| Ammoniak (konz.) bis | pH = 10 | pH = 10 | pH = 10 | pH = 10 | pH = 10 |
| Wasser | ad 100 g | ad 100 g | ad 100 g | ad 100 g | ad 100 g |
| Viskosität [Pa·s] 20 °C | 2,7 | 2,15 | 3,5 | 1,75 | 2,1 |

Herstellung: Die Fettsäure wurde mit Ammoniak (konz.) in Gegenwart von wenig Wasser (ca. 15 g) und 1,2-Propylenglycol verseift, dann Texapon N, Dehyton K, die Mischung aus Arlypon F, K 20, DTE und DTEE und Parfümöl zugegeben, schließlich Farbstofflösung, Stabilisatorlösung und das restliche Wasser der Reihe nach eingemischt. Die Mischung wurde mit Ammoniak (konz.) auf pH = 10 eingestellt.

Die Messung der Viskosität erfolgte bei 20 °C mit einem Brookfield Rotationsriskosimeter, Type RTV, Spindel 4, 4 Umdrehungen pro Minute).

### Oxidationsmittel-Zubereitung (B)

| | |
|---|---|
| Wasserstoffperoxid (50 %ig in H₂O) | 12,0 g |
| Acrylat-Methacrylsäure-Copolymer, 25 %ige wäßrige Dispersion, (Latekoll ^{(R)}D) | 2,0 g |
| Texapon N (3) | 2,0 g |
| Kollagenhydrolysat (Lexein ^{(R)}) | 0,5 g |
| 1-Hydroxyethan-1,1-diphosphonsäure | 0,18 g |
| Na₂H₄P₂O₇ | 0,03 g |
| Ammoniak, konz. | bis pH = 4 |
| Wasser | ad 100 g |

Herstellung: Die Komponenten wurden der Reihe nach im Wasser gelöst.

In der folgenden Tabelle II sind die Viskositäten der durch Vermischen der Oxidationsfarbstoff-Zubereitungen 1 bis 5 (A) (gemäß Tabelle I) mit der Oxidationsmittel-Zubereitung (B) im Gewichtsverhältnis 1:1 erhaltenen Färbeansätze aufgeführt:

**Tabelle II**

| Färbeansatz: A:B = 1:1 | auf Basis der Haarfärbemittel-Zubereitung | | | | |
|---|---|---|---|---|---|
| | 1 | 2 | 3 | 4 | 5 |
| Viskosität [Pa·s] 20 °C | 20,6 | 28,0 | 29,0 | 13,4 | 23,7 |

## Patentansprüche

1. Haarfärbemittel, bestehend aus einer fließfähigen, wäßrigen Oxidationsfarbstoffzubereitung (A) und einer fließfähigen, wäßrigen Oxidationsmittelzubereitung (B), die unmittelbar vor dem Aufbringen auf das Haar miteinander im Gewichtsverhältnis A:B = 1:2 bis 2:1 zu einem gelförmigen Färbeansatz vermischt werden, dadurch gekennzeichnet, daß die Oxidationsfarbstoffzubereitung (A) als Trägerkomponenten
0,5 bis 5 Gew.-% einer flüssigen Fettsäure mit 16 bis 22 C-Atomen in Form einer wasserlöslichen Seife,
1 bis 5 Gew.-% eines Polyols mit 2 bis 6 C-Atomen,
0,5 bis 5 Gew.-% eines wasserlöslichen synthetischen Tensids und zur Verdickung
1 bis 10 Gew.-% eines Anlagerungsproduktes von 1 bis 5 Mol Ethylenoxid an einen linearen Fettalkohol mit 12 bis 22 C-Atomen und/oder
1 bis 6 Gew.-% eines Anlagerungsproduktes von 1 bis 4 Mol Ethylenoxid an ein lineares Fettalkylamin mit 12 bis 22 C-Atomen und gegebenenfalls
0 bis 10 Gew.-% einer Verbindung der Formel I
R¹-(OC₂H₄)ₓ-A-(C₂H₄O)_{y}-R² (I)
in der R¹ und R² lineare Alkyl- oder Alkenylgruppen mit 12 bis 22 C-Atomen, x und y = 0 oder 1 und A ein Sauerstoffatom oder eine Gruppe HO-C₂H₄-N ist, enthält.

2. Haarfärbemittel gemäß Anspruch 1, dadurch gekennzeichnet, daß die Oxidationsmittelzubereitung (B)
3 bis 10 Gew.-% Wasserstoffperoxid
0,1 bis 5 Gew.-% eines wasserlöslichen, synthetischen Tensids und
1 bis 5 Gew.-% eines dispergierten Acrylsäure- und/oder Methacryl
säure-Polymerisats oder Copolymerisats
enthält.

3. Haarfärbemittel nach Anspruch 1 und 2, dadurch gekennzeichnet, daß die Oxidationsfarbstoffzubereitung (A) Ammoniak und Puffersalze zur Einstellung eines pH-Wertes von 8 bis 10 und die Oxidationsmittelzubereitung (B) Komplexbildner und Puffersalze zur Einstellung eines pH-Wertes von 2 bis 5 enthält.

4. Haarfärbemittel nach Anspruch 1, dadurch gekennzeichnet, daß die Oxidationsfarbstoffzubereitung (A) als synthetisches Tensid ein Gemisch aus einem Alkylethersulfat-Tensid und einem amphoteren oder zwitterionischen Tensid enthält.

## Claims

1. Hair dyes consisting of a fluid aqueous oxidation dye preparation (A) and a fluid aqueous oxidizing agent preparation (B), which are mixed together in a ratio by weight of A:B of 1:2 to 2:1 to form a gel-like dye immediately before application to the hair, characterized in that the oxidation dye preparation (A) contains as carrier components
0.5 to 5% by weight of a liquid fatty acid containing 16 to 22 carbon atoms in the form of a water-soluble soap,
1 to 5% by weight of a polyol containing 2 to 6 carbon atoms,
0.5 to 5% by weight of a water-soluble synthetic surfactant
and, for thickening,
1 to 10% by weight of an adduct of 1 to 5 mol ethylene oxide with a linear C₁₂₋₂₂ fatty alcohol and/or
1 to 6% by weight of an adduct of 1 to 4 mol ethylene oxide with a linear C₁₂₋₂₂ fatty alkyl amine and, optionally,
0 to 10% by weight of a compound corresponding to formula (I)
R¹-(OC₂H₄)ₓ-A-(C₂H₄O)_{y}-R² (I)
in which R¹ and R² are linear C₁₂₋₂₂ alkyl or alkenyl groups, x and y = 0 or 1 and A is an oxygen atom or a group HO-C₂H₄-N〈 .

2. Hair dyes as claimed in claim 1, characterized in that the oxidizing agent preparation (B) contains
3 to 10% by weight hydrogen peroxide
0.1 to 5% by weight of a water-soluble synthetic surfactant and
1 to 5% by weight of a dispersed acrylic acid and/or methacrylic acid polymer or copolymer.

3. Hair dyes as claimed in claims 1 and 2, characterized in that the oxidation dye preparation (A) contains ammonia and buffer salts to establish a pH value of 8 to 10 and the oxidizing agent preparation (B) contains complexing agents and buffer salts to establish a pH value of 2 to 5.

4. Hair dyes as claimed in claim 1, characterized in that the oxidation dye preparation (A) contains a mixture of an alkyl ether sulfate surfactant and an amphoteric or zwitterionic surfactant as the synthetic surfactant.

## Revendications

1. Teinture pour les cheveux formée d'une composition de colorant par oxydation aqueuse, fluide (A), et d'une composition d'agent d'oxydation, aqueuse, fluide (B) qui sont mélangées immédiatement avant l'application sur les cheveux l'une avec l'autre, dans un rapport pondéral A : B = 1 : 2 à 2 : 1 pour constituer une préparation de coloration gélifiée, caractérisée en ce que la composition de colorant par oxydation (A) contient en tant que composants support :
0,5 à 5 % en poids d'un acide gras liquide ayant de 16 à 22 atomes de carbone sous la forme d'un savon soluble dans l'eau,
1 à 5 % en poids d'un polyol avec 2 à 6 atomes de carbone,
0,5 à 5 % en poids d'un agent tensioactif synthétique, soluble dans l'eau et en vue de l'épaississement,
1 à 10 % en poids d'un produit d'addition de 1 à 5 mol d'oxyde d'éthylène sur un alcool gras linéaire ayant de 12 à 22 atomes de carbone et/ou,
1 à 6 % en poids d'un produit d'addition de 1 à 4 mol d'oxyde d'éthylène sur une alcoylamine grasse linéaire, ayant de 12 à 22 atomes de carbone,
et le cas échéant
0 à 10 % en poids d'un composé de formule (I) :
R¹-(OC₂H₄)ₓ-A-(C₂H₄O)_{y}-R² (I)
dans laquelle R¹ et R² sont des radicaux alcoyle ou alcényle linéaires ayant de 12 à 22 atomes de carbone, x et y = 0 ou 1 et A est un atome d'oxylène ou un groupe HO-C₂H₄-N.

2. Teinture pour cheveux selon la revendication 1, caractérisée en ce que la composition d'agent d'oxydation (B) renferme :
3 à 10 % en poids de peroxyde d'hydrogène,
0,1 à 5 % en poids d'un agent tensioactif synthétique soluble dans l'eau, et
1 à 5 % en poids d'un polymérisat ou d'un copolymérisat d'acide acrylique et /ou d'acide méthacrylique dispersé.

3. Teinture pour les cheveux selon la revendication 1 ou 2, caractérisée en ce que la composition de colorant par oxydation (A) contient de l'ammoniaque et des sels tampons pour ajuster la valeur du pH de 8 à 10 et la composition d'agent d'oxydation (B) renferme un agent complexant et des sels tampon pour ajuster la valeur du pH de 2 à 5 .

4. Teinture pour les cheveux selon la revendication 1, caractérisée en ce que la composition de colorant par oxydation (A) renferme comme agent tensioactif synthétique un mélange à base d'agent tensioactif formé de sulfate d'éther d'alcoyle et d'un agent tensioactif amphotère ou zwitterionique.
